Europäisches Patentamt

European Patent Office

Office européen des brevets

(11) Veröffentlichungsnummer: **0 123 316 B1**

(12) **EUROPÄISCHE PATENTSCHRIFT**

(45) Veröffentlichungstag der Patentschrift: **15.01.92**

(21) Anmeldenummer: **84104642.8**

(22) Anmeldetag: **25.04.84**

(51) Int. Cl.⁵: **G01N 33/12**, B01D 57/00, C12M 1/12

(54) **Vorrichtung und Verfahren zur Erzeugung bzw. Freisetzung und Abtrennung von Substanzen oder partikulären Gebilden aus flüssiger, plastischer oder fester Materie und Verwendung der Vorrichtung.**

(30) Priorität: **25.04.83 DE 3314937**

(43) Veröffentlichungstag der Anmeldung:
**31.10.84 Patentblatt 84/44**

(45) Bekanntmachung des Hinweises auf die Patenterteilung:
**15.01.92 Patentblatt 92/03**

(84) Benannte Vertragsstaaten:
**AT BE CH DE FR GB IT LI NL SE**

(56) Entgegenhaltungen:
**EP-A- 0 059 403**
**DE-B- 2 746 257**

(73) Patentinhaber: **A/S N. Foss Electric**
**Slangerupgade 69**
**DK-3400 Hilleroed(DK)**

(72) Erfinder: **Pfeiffer, Gottfried, Prof. Dr.**
**Hufeisenstrasse 13**
**W-8057 Eching(DE)**

(74) Vertreter: **Hansen, Bernd, Dr.rer.nat. et al**
**Hoffmann, Eitle & Partner Patentanwälte Arabellastrasse 4 Postfach 81 04 20**
**W-8000 München 81(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Vorrichtung und ein Verfahren zur Erzeugung bzw. Freisetzung und Abtrennung von Substanzen oder partikulären Gebilden aus flüssiger, plastischer oder fester Materie und Verwendung der Vorrichtung.

Auf den Gebieten der analytischen Untersuchung sowie der Gewinnung von Substanzen oder partikulären Gebilden durch deren Erzeugung und/oder deren Isolierung aus einem flüssigen bzw plastischen oder festen Material, z.B. einem biologischen Material, besteht ein Bedarf nach einer Vorrichtung sowie einem Verfahren, mit deren Hilfe sich die Erzeugung bzw. Freisetzung von Substanzen und deren Abtrennung möglichst schnell durchführen lässt. Insbesondere sind solche Vorrichtungen von Interesse, die eine Automatisierung der dabei durchzuführenden Teilschritte erlauben. Sofern es möglich ist, die Schritte der Erzeugung bzw. Freisetzung einer Substanz bzw. eines partikulären Gebildes und dessen Abtrennung vom restlichen Gemisch in einer einzigen Vorrichtung durchzuführen und dabei die Verfahrensparameter in den einzelnen Teilschritten sowie die Zugabe von Beschickungsgut, die Be und Entgasung, aber auch den zwischen den einzelnen Untersuchungen bzw. Experimenten liegenden Nachspül oder Waschvorgang zu automatisieren, lassen sich derartige Verfahren in Serie durchführen. Auf diese Weise ist es möglich, nennenswerte Personalkosten einzusparen, sowie das jeweilige Verfahren im Hinblick auf seinen Anwendungszweck zu optimieren.

Aufgabe der vorliegenden Erfindung ist es, eine Vorrichtung und ein Verfahren zur Erzeugung bzw. Freisetzung und Abtrennung von Substanzen oder partikulären Gebilden aus flüssiger, plastischer oder fester Materie zur Verfügung zu stellen.

Diese Aufgabe wird gemäss der Erfindung durch eine Vorrichtung der vorstehend genannten Art gelöst, die gekennzeichnet ist durch einen sich nach unten verjüngenden Reaktor , welcher in seinem unteren Teil ein Ablassventil aufweist und über ein sich konisch erweiterndes Teil mit einer nachgeschalteten Separiervorrichtung verbunden ist, wobei die Separiervorrichtung einen zylindrischen Anschlussstutzen umfasst, der Halteringe zur Halterung von einem oder mehreren Filtern bzw. Sieben aufnimmt, und sich in einem lösbaren Kontakt mit dem über und/oder unter dem Anschlussstutzen liegenden, sich konisch erweiternden Teil befindet.

Im weiteren wird ein Verfahren der vorstehend genannten Art zur Verfügung gestellt, bei welchem unter Verwendung der erfindungsgemässen Vorrichtung das gelöste plastische oder feste Material zusammen mit einer oder mehreren Flüssigkeiten in einem Reaktor einer chemischen und/oder physikalischen Behandlung unterworfen, das erhaltene Gemisch über das sich im unteren Teil des Reaktors befindliche Ablassventil einer Separiervorrichtung zugeführt, in welcher mit Hilfe eines oder mehrerer Filter eine Auftrennung desselben in eine flüssige und eine oder mehrere feste Phasen vorgenommen wird, die sich gegebenenfalls durch die Grösse ihrer korpuskulären Bestandteile voneinander unterscheiden. Die Anordnung des Filtersystems der Trennvorrichtung kann deshalb ebenso auch darauf abgestellt sein, dass Komponenten von einer bestimmten Korpuskelgrösse oder Molekülgrösse an in der flüssigen Phase enthalten sind und diese Gegenstand der weiteren Untersuchung oder Behandlung bilden.

Die Erfindung umfasst insbesondere ein Verfahren unter Anwendung der erfindungsgemässen Vorrichtung zur Untersuchung von Schweinefleisch auf Trichinen.

Die Verwendung der erfindungsgemässen Vorrichtung erfolgt auf zahlreichen Gebieten der Analytik sowie der Gewinnung und Isolierung von Substanzen oder partikulären Gebilden, sowie nicht zuletzt bei technologischen Studien an kleinsten Rohstoffmengen und Verfahrensansätzen.

Die erfindungsgemässe Vorrichtung ist in diesem Rahmen auch insofern von einem besonderen Interesse, als sie Verfahrensweisen ermöglicht, bei denen sich in echten und kolloidalen Lösungen sowie in Dispersionen und Emulsionen durch Ausfällen, Koagulieren und Agglomerieren oder durch Koaleszieren spezifische Aggregate erzeugen, abtrennen und gegebenenfalls mikroskopisch untersuchen lassen. Bei diesen Anwendungsfällen kommt es z.B. häufig darauf an, die Lösungen, Dispersionen und Emulsionen vor, während oder nach der Erzeugung der Aggregate chemisch oder z.B. mittels eines Adsorptionsmittels, wie Tierkohle, physikalisch zu entfärben. Ebenso häufig kommt es aber auch darauf an, bestimmte Komponenten der Lösungen, Dispersionen, Emulsionen vor, während oder nach der Erzeugung der Aggregate mit Farbstoffen oder radioaktiven Substanzen zu markieren oder mit anderen Stoffen zu beaufschlagen. Bei serologischen Studien kommt es bei zahlreichen Fragestellungen auf eine Agglomeration und Abtrennung von Antigen-Antikörper-Assoziatiaten und/oder die Gewinnung einer von solchen Assoziaten befreiten flüssigen Phase an.

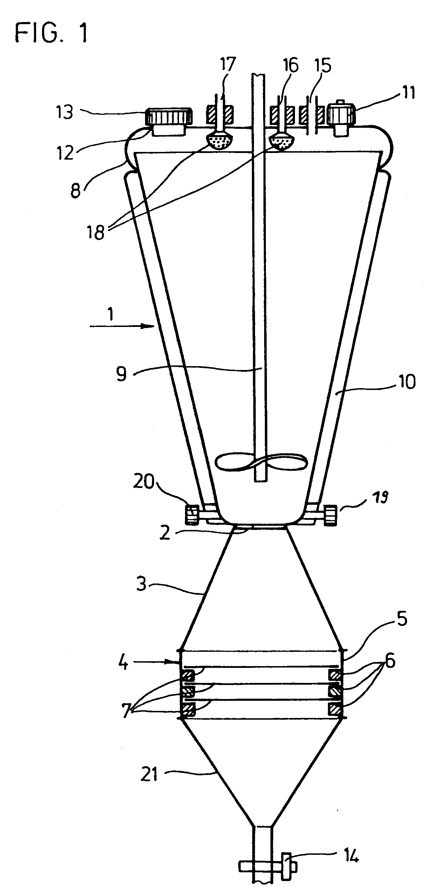

Fig. 1 gibt eine Darstellung der erfindungsgemässen Vorrichtung wieder. Der Reaktor 1, der sich vorzugsweise nach unten trichterförmig bzw. konisch verjüngt, dient zur Aufnahme der plastischen oder festen Materie. Durch einen abnehmbaren Deckelteil 8 lässt sich der Reaktor 1 flüssigkeits- bzw. gasdicht abschliessen. Ein im Deckelteil 8 befindliches Ventil 11 dient dem Druckausgleich oder der Zufuhr von z.B. Pressluft. Der Deckelteil 8 umfasst ausserdem eine Öffnung 12 zum Beschicken des Reaktors mit Probengut. Diese Öffnung wird vorzugsweise mit einem Stopfen 13 mit Bajonettverschluss vakuum und druckdicht verschlossen. Ausserdem ragt in den Deckelteil zentrisch der Schaft einer Zerkleinerungsvorrichtung 9, die vorzugsweise ein Drehmesser darstellt, der vorzugsweise stufenlos auf Umdrehungen bis 3x10⁴/min. schaltbar ist oder durch manuelle Einzelschaltung bedient wird.

Die Eintrittsventile 15, 16, 17 sind vorzugsweise druckdicht verschliessbar und können mit Sprühdüsen 18 ausgestattet sein.

Der mittlere Teil des Reaktors 1 wird flüssigkeits- bzw. gasdicht von einem Mantel 10 umgeben, mittels welchem über eine zirkulierende Flüssigkeit oder ein entsprechendes Gas der Reaktorwand Wärme zugeführt oder entzogen werden kann. Bevorzugt ist eine Temperierung im Bereich von -60°C bis 250°C, insbesondere im Bereich von -10 bis 60°C. Unterhalb dieses Mantels befindet sich eine Schrauböffnung 19 bzw. 20, durch die mit einem Temperaturfühler bzw. einer ionensensitiven Elektrode oder einem Leitfähigkeitsmesser oder einem Viskositätsmesser oder einem anderen Messinstrument eingegangen werden kann.

Der sich nach unten verjüngende Reaktor 1 besitzt in seinem Bodenteil ein Ablassventil 2, das eine Verbindung des Reaktors 1 mit der nachfolgenden Separiervorrichtung 4 darstellt. Das Ablassventil 2 kann z.B. ein Magnetventil, ein Zwei-Weg-Hahn oder ein anderes geeignetes Ventil darstellen.

Der untere Teil des Reaktors 1 mit dem Ablassventil 2 setzt sich in einem von ihm über einen Bajonett oder Gewindeverschluss lösbaren, sich konisch erweiternden Teil 3 fort, welche mit der nachgeschalteten Separiervorrichtung 4 verbunden ist.

Die Separiervorrichtung 4 besteht aus einem zylindrischen Anschlussstutzen 5. Dieser Anschlussstutzen 5 nimmt Halterungen 6 auf, die zur Halterung von einem oder mehreren Filtern bzw. Sieben 7 dienen. Der Anschlussstutzen 5 befindet sich in fester Verbindung entweder mit Teil 3 oder Teil 21 und ist in einem lösbaren Kontakt, z.B. über einen Gewinde oder Bajonettverschluss, entsprechend mit Teil 21 oder Teil 3; oder die Separiervorrichtung 4 ist in lösbarem Kontakt sowohl mit Teil 3 und Teil 21.

Die Separiervorrichtung 4 ist an ihrem unteren Ende mit einem Ablassventil 14 ausgestattet.

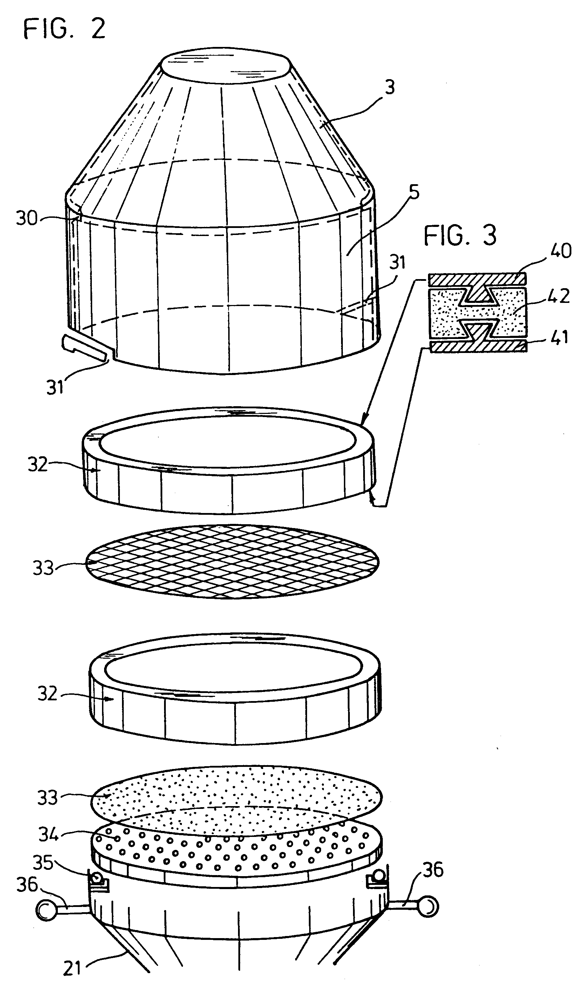

Fig. 2 stellt einen bevorzugten Aufbau für die Separiervorrichtung 4 dar. Danach befindet sich der Anschlussstutzen 5 in festem Kontakt mit Teil 3. 30 stellt eine Anpressfläche für eine obere Gummidichtung dar. 31 ist eine Einrastung für einen Bajonettverschluss. Der Anschlussstutzen 5 nimmt Abstandhalteringe 32, gegebenenfalls mit eingefügter Dichtung, auf. Die Filter 33 befinden sich jeweils zwischen den Abstandhalteringen 32. Eine Membranträger-Sieblochscheibe 34 dient als Unterstützung des Filters mit der feinsten Perforation, und ist auf einem O-Ring 35 gelagert. Einrasterstift 36 dient zum Einrasten mittels Bajonettverschluss. Der Anschlussstutzen 5 ist somit in lösbarem Kontakt mit Teil 21.

Fig. 3 ist eine Querschnittsdarstellung eines bevorzugten Abstandhalteringes, bestehend aus den profilierten Ringen 41 und der dazwischenliegenden Gummidichtung 42.

Mit Hilfe der erfindungsgemässen Vorrichtung lässt sich ein Verfahren zur Erzeugung bzw. Freisetzung und Abtrennung von Substanzen oder partikulären Gebilden aus flüssiger bzw. plastischer oder fester Materie durchführen. Zu diesem Zweck führt man in den Reaktor 1 entweder nur flüssiges bzw. das plastische oder feste Material zusammen mit einer Flüssigkeit ein und unterwirft es einer chemischen und/oder physikalischen Behandlung.

Die eingeführte Substanz kann für eine oder mehrere der freizusetzenden Substanzen ein Lösungsmittel darstellen, wie z.B. Wasser, Pufferlösung oder ein organisches Lösungsmittel.

Die chemische Behandlung kann z.B. eine Behandlung mit einer gegebenenfalls starken Säure oder Base, einer farbgebenden oder entfärbenden Substanz bzw. Lösung, oder einem Gas darstellen oder ein enzymatisches Aufschlussverfahren mit Hilfe einer gepufferten Enzymlösung oder eine serologische Reaktion sein. Im Falle der enzymatischen Behandlung des genannten Materials stellt die im Reaktor zugegebene Flüssigkeit vorzugsweise Wasser oder eine Pufferlösung dar. Als Enzyme für die enzymatische Behandlung eignen sich besonders Hydrolasen, insbesondere proteolytische Enzyme, darüber hinaus jedoch auch Oxidasen, Reduktasen, Transaminasen und eine Vielzahl anderer Enzyme.

Je nach der Art der chemischen Behandlung kann das sich im Reaktor befindliche Gemisch auf die geeignete Temperatur gebracht und auf derselben thermostatisiert werden.

Die physikalische Behandlung des genannten Materials erfolgt besonders vorteilhaft durch eine Kälteoder Hitzebehandlung, z.B. zur Erzielung einer Koagulation, Kristallisation oder von Schmelzvorgängen, oder Zerkleinerung desselben. Für die Zerkleinerung verwendet man eine geeignete Zerkleinerungsvorrichtung, so z.B. vorzugsweise ein Drehmesser. Geeignet ist aber auch ein an seiner Oberfläche gegebenenfalls profilierter Stator, innerhalb dessen ein von einem vorzugsweise ausserhalb des Stators gelegenen Motor angetriebener Rotor mit entsprechend profilierter Oberfläche gedreht wird. Die Zerkleinerungsvorrichtung wird vorzugsweise stufenlos bei bis zu $3 \times 10^4$ UpM betrieben. Nach weiteren Ausführungsformen erfolgt der Antrieb der Zerkleinerungsvorrichtung durch manuelle Einzelschaltung oder nach einem vorwählbaren Zeitschaltprogramm automatisch.

Nach einer weiteren Modifikation kann eine physikalische Behandlung unter gegebenenfalls Zerkleinerung oder Agglomeration des genannten Materials mittels Ultrabeschallung erfolgen. In diesem Falle erfolgt die Ultrabeschallung vorzugsweise über einen durch den Deckelteil 8 oder die Wandung des Reaktors eingeführten Schaft 9.

Sofern zur Behandlung des genannten Materials keine Zerkleinerungsvorrichtung erforderlich ist, kann durch den Deckelteil 8 oder durch die Wandung des Reaktors auch eine Rührvorrichtung eingeführt werden.

Besonders vorteilhaft ist es, eine physikalische Behandlung mit einer chemischen zu kombinieren, so z.B. zunächst eine physikalische Zerkleinerung des plastischen oder festen Materiales herbeizuführen, das anschliessend z.B. durch enzymatische Behandlung weiter abgebaut wird.

Der oder die Filter sind so abgedichtet, dass es bei der Passage des flüssigen Anteils des Ansatzes durch den oder die Filter für die Flüssigkeit nur einen Weg nach aussen gibt, nämlich den durch die Siebbzw. Porenöffnungen des oder der Filter.

Das nach der chemischen und/oder physikalischen Behandlung erhaltene Gemisch wird über das Ablassventil 2 in die Separiervorrichtung 4 gebracht, in welcher es mit Hilfe eines oder mehrerer Filter in eine flüssige und eine oder mehrere feste Phasen aufgetrennt wird.

Die bei der Auftrennung erhaltene flüssige Phase kann dabei eine Lösung, Dispersion oder eine Emulsion darstellen.

Die Auftrennung in der Separiervorrichtung, die über eines oder mehrere Filter, Siebe oder Siebmembranen erfolgt, kann unter Druckeinwirkung über das Ventil 11 oder durch Anlegen eines Vakuums im unteren Teil der Separiervorrichtung erfolgen.

Die Auftrennung in der Separiervorrichtung wird vorzugsweise über ein, zwei oder drei Filter, Siebe oder Siebmembranen durchgeführt, wobei bei Verwendung von zwei oder mehr Filtern etc. Partikel unterschiedlicher Grösse auf denselben zurückgehalten werden können. Die Porosität der einzelnen Filter nimmt dabei in Durchflussrichtung ab.

Auf diese Weise erhält man auf den Filtern abgesonderte feste Phasen aus korpuskulären Bestandteilen, die sich hinsichtlich des Einzelvolumens und gegebenenfalls des Gesamtvolumens der korpuskulären Bestandteile in Abhängigkeit vom Querschnitt der Poren bzw. Sieböffnungen voneinander unterscheiden.

Die Auftrennung in der Separiervorrichtung erfolgt nach einer Modifikation gemäss der Erfindung über zwei oder drei verschiedene Filter. Das dem Ablassventil am nächsten gelegene Filter oder Sieb besteht vorzugsweise aus einem zu einer glatten Oberfläche verschweissten Kunststoff- oder Metallgeflecht mit Sieböffnungen, die insbesondere rautenförmig sind und einen Längsdurchmesser von 1 bis 2 mm und einen Querdurchmesser von 0,7 bis 1,4 mm aufweisen.

Das in Durchflussrichtung darauffolgende zweite Sieb, ist vorzugsweise ein Sieb mit einer glatten Oberfläche und Sieböffnungen mit einem Durchmesser von 0,18 bis 0,6 mm und vorzugsweise 0,4 mm.

Das dritte Sieb ist ein von einer Sieblochscheibe mit Sieböffnungen vom Durchmesser 0,3 bis 1 mm getragenes, vorzugsweise aus Polycarbonat bestehendes transparentes Membransieb, dessen Sieböffnungen einen Durchmesser von 8 bis 30 $\mu$m und vorzugsweise 12 $\mu$m aufweisen.

Von den vorstehend genannten drei Filtern bzw. Sieben kann nach einer Ausführungsform gemäss der Erfindung das zweite weggelassen werden, so dass nur eine Kombination aus zwei der vorstehend genannten Filter verwendet wird.

Bevorzugt wird gemäss der Erfindung zur Auftrennung des zerkleinerten Gemisches ein Satz aus mehreren transparenten Membransieben mit Sieböffnungen vom Durchmesser bis zu 180 $\mu$m, deren Porengrösse in Durchflussrichtung abnimmt, verwendet.

Ein besonderer Vorteil des erfindungsgemässen Verfahrens unter Anwendung der Vorrichtung gemäss der Erfindung besteht darin, dass mit geringem Aufwand entweder manuell oder automatisch ein Nachspülgang durchgeführt werden kann. Ausserdem ist es möglich, nach jeder Bestimmungsmethode die erfindungsgemässe Vorrichtung leicht auf manuelle oder automatische Weise zu reinigen.

Das erfindungsgemässe Verfahren kann zur Erzeugung bzw. Freisetzung und Abtrennung von Substanzen bzw. korpuskulären Gebilden auf vielen Gebieten angewendet werden. Da gemäss der Erfindung eine Kombination der Verfahrensschritte Erzeugung bzw. Freisetzung und Abtrennung erfolgt, können auf diese Weise besonders leicht analytisch zu bestimmende oder zu untersuchende Substanzen oder Partikel in einer komplexen Materie erzeugt bzw. aus einer solchen freigesetzt oder desaggregiert und aufgetrennt werden. Ausserdem ist das erfindungsgemässe Verfahren geeignet zur Isolierung von Substanzen oder Partikeln aus komplexer Materie.

So lässt sich z.B. das erfindungsgemässe Verfahren besonders gut anwenden zur Freisetzung und Abtrennung von beispielsweise Parasiten oder Parasiteneiern bzw. nicht verdauten Partikeln aus Stuhlproben oder Abwässern, Stärkekörnern aus rohen Kartoffeln, etc., Gewürzen aus Lebensmitteln, Oxalatkristallen aus Baumrinden, Blütenpollen aus Pollenkonglomeraten Bakterien und Viren aus Wurstgut, von Zellorganellen, wie z.B. Nuclei, Mitochondrien, etc., aus biologischem Material. Darüber hinaus können aus biologischem Material mit Hilfe des erfindungsgemässen Verfahrens auch Enzyme, Metabolite, diverse Makromoleküle isoliert bzw. nach dem Prinzip der Ultrafiltration angereichert respektive entfernt werden. Für jeden der beispielhaft genannten Anwendungsfälle sind Filter und Filtermembranen verschiedenster Porosität im Handel erhältlich und dem jeweiligen Zweck entsprechend in der Separiervorrichtung spezifisch kombinierbar.

Schliesslich eignet sich das erfindungsgemässe Verfahren auch zur Aufbereitung von Bodenproben und Freisetzung sowie Abtrennung der zu untersuchenden Substanzen.

Unter den vorstehend genannten Methoden der analytischen Untersuchung von Substanzen oder korpuskulären Gebilden aus biologischem Material ist insbesondere die Bestimmung von Trichinen in Schweinefleisch von besonderem Interesse.

Zur näheren Erläuterung des erfindungsgemässen Verfahrens unter Anwendung der Vorrichtung gemäss der Erfindung wird nachstehend ein verbessertes Verfahren zur Bestimmung von Trichinen in Schweinefleisch beschrieben. Die Erfindung ist jedoch nicht auf diese Ausführungsform beschränkt.

Verfahren zur Bestimmung von Trichinen in Schweinefleisch

I) Vorrichtung

Diese besteht aus der erfindungsgemässen Apparatur, bestehend aus einem Reaktorteil mit angeschlossener Separiervorrichtung mit einer Kombination aus vorzugsweise vier Siebscheiben vom Durchmesser 47 bis 50 mm. Zur bevorzugten Beschaffenheit der Siebe unter "III. Bevorzugte Verfahren zur Bestimmung von Trichinen in Schweinefleisch mit der erfindungsgemässen Apparatur".

Der Auslauf dieser Apparatur ragt vakuumdicht in einen mit einer Wasserstrahlpumpe evakuierten, in seiner Funktion einer Laborsaugflasche vergleichbaren Vakuumbehälter mit Ablasshahn am Boden zur Entfernung von flüssiger Phase. Durch Öffnen des Ablassventils 2 und des Ablasshahnes der Separiervorrichtung 4 wird der Ansatz im Reaktor über Vakuum gebracht, so dass sein flüssiger Anteil bei geöffnetem Ventil 11 oder geöffneter Deckelöffnung 12 die Trennvorrichtung passiert.

Der Zwischenraum zwischen Reaktorwand und Mantel 10 ist über einen Schlauch zur Zuleitung und einen Schlauch zur Ableitung von thermostatisiertem Wasser von 46°C mit der Umwälzpumpe eines Wasserbades verbunden. Oberhalb der Apparatur sind drei Vorratsbehälter abgestellt, deren Auslauf jeweils über eine Dosierpumpe und mit Schläuchen mit den am Deckel 8 angebrachten, jeweils druckdicht verschliessbaren Eintrittsventilen 15, 16, 17 verbunden ist. Das für die Zufuhr von 0,75%iger HCl-Lösung bestimmte, dem Schaft des Drehmessers am nächsten gelagerte Einlassventil 16 und das Einlassventil 17 münden jeweils in eine Sprühdüse, mit der am Deckel oder an der Reaktorwand haftenden Grobbestandteile hinabgespült werden.

Bei den genannten Vorratsbehältern handelt es sich um einen 2 bis 5 1-Vorratsbehälter für 5%ige Pepsinlösung, eingestellt mit HCl auf pH 5,5, einen 10 1-Vorratsbehälter für 0,75%ige HCl und einen 10 1-Vorratsbehälter für Leitungswasser vom pH 1,1.

Ausserdem werden eine Glasplatte 70x80x3 mm und eine Glasplatte 60x60x3 mm, letztere mit im Abstand von 3 mm eingeritzten parallelen Linien sowie ein Mikroskop mit 20- und 40-facher Vergrösserung und abblendbarem Kondensor benötigt.

II) Materialien

a) Probenmaterial

Ein oder mehrere walnussgrosse Muskelstücke aus dem Zwerchfellpfeiler von Schlachtschweinen oder anderes trichinenverdächtiges Untersuchungsmaterial.

b) Reagentien

1) Pepsin, 30.000 E/g
2) Konzentrierte Salzsäure
3) Octanol

c) Lösungen

1) 5%ige wässrige Pepsinlösung
2) 0.75%ige und 0,5%ige HCl-Lösung
3) Leitungswasser, eingestellt auf pH 1,1

III. Bevorzugte Verfahren zur Bestimmung von Trichinen im Schweinefleisch mit der erfindungsgemässen Apparatur

Die im folgenden geschilderten Verfahrenweisen haben gegenüber dem Stand der analytischen Chemotechnik drei entscheidende Vorteile gemeinsam:

1. die sehr rasche mechanische und chemische Zerkleinerung,

2. die sehr rasche Abtrennung von Trichinen aus dem Ansatz,

3. die sehr rasche, einfache und sichere Nachweisbarkeit der Trichinen auf dem transparenten Membransieb.

Erster bevorzugtes Verfahren

(a) Je 1 g Zwerchfellmuskulatur von 25 bis 45 Schweinen werden zu einer Sammelprobe zusammengefasst und durch die Öffnung 12 im Deckel in den Reaktor gegeben, der anschliessend oder zuvor mit 100 ml 5%iger wässriger Pepsinlösung vom pH 5,5, bezogen auf das Gewicht der Sammelprobe, beschickt und dessen Wand durch Zirkulieren einer Flüssigkeit zwischen ihr und ihrem Mantel auf 46°C gebracht wird. Danach wird die Öffnung 12 geschlossen.

(b) Die Sammelprobe wird nun mit abgestumpftem Drehmesser 9 solange zerkleinert, bis der nichtbindegewebehaltige Teil der Skelettmuskulatur überwiegend gelöst oder grob dispers vorliegt. Dieser Zerkleinerungsvorgang dauert bei einer Messerdrehzahl von 7.000 bis 12.000 UpM insgesamt 1 bis 3 Minuten. Während der mechanischen Vorzerkleinerung werden an der Reaktorwand oder am Deckel 8 haftende Grobbestandteile der Sammelprobe von Anfang an hinabgespült. Das Hinabspülen erfolgt durch Einleiten einer 0,75%igen HCl-Lösung unter Druck durch eine durch den Deckel einmündende Zuleitung 16 zu einer Sprühdüse 18.

(c) Im weiteren Prozess wird die Drehgeschwindigkeit des Messers auf 50 bis 5 %, vorzugsweise 10% der Anfangsdrehzahl verringert und solange kontinuierlich 0,75%ige HCl zugeführt, bis sich im Ansatz vorzugsweise ein pH von 1,1, jedenfalls aber 0,8 bis 2,2 einstellt. Dies wird volumetrisch dadurch erreicht, dass insgesamt 400 ml einer 0,75%ige HCl-Lösung zugeführt werden.

(d) Anschliessend wird der Ansatz vorzugsweise 8 Minuten bzw. solange gerührt, bis der Hauptteil der Trichinen aus ihrer Verkapselung befreit, nur noch einige wenige bindegewebige oder Blutgefässen entstammende Partikel in etwa Kümmelkorn-Grösse vorliegen und der flüssige Teil des Ansatzes mit seinen gelösten, dispergierten bzw. kleiner als 12 $\mu$m grossen korpuskulären Bestandteilen auch durch das unterste Sieb der Trennvorrichtung, d.h. vorzugsweise durch ein transparentes Membransieb mit Porenöffnungen vom Durchmesser 12 $\mu$m passierbar ist. Der im Ansatz während dieser mechanischen und enzymatischen Zerkleinerung geringgradig entstandene Schaum wird vor der Abtrennung der Trichinen vorzugsweise gebrochen. Dies geschieht dadurch, dass dem Ansatz unmittelbar vor dem Abschalten des Drehmessers ca. 0,5 ml Octanol aus einer Tropfpipette durch die Deckelöffnung 12 zugegeben werden.

(e) Die Abtrennung der Trichinen und der unter (d) genannten Bindegewebs- und/oder Blutgaefässfragmente erfolgt vorzugsweise über Vakuum, indem die Ventile 11, 2 und 14 geöffnet werden und der flüssige Teil des Ansatzes in der Durchflussrichtung der Reihe nach vier Siebfilter passiert, die folgende Merkmale aufweisen:

Nr. 1) ist ein vorzugsweise zu einer glatten Oberfläche verschweisstes Kunststoff- oder Metallgeflecht mit vorzugsweise rautenförmigen Sieböffnungen mit einem Längsdurchmesser von 1,0 bis 2.0 mm und vorzugsweise 1,5 mm und einem Querschnitt von 0,7 bis 1,4 mm und vorzugsweise 1 mm.

Nr. 2) ist ein vorzugsweise zu einer glatten Oberfläche verschweisstes Kunststoffoder Metallgeflecht mit Sieböffnungen mit einem Durchmesser von 0,3 bis 1,2 mm und vorzugsweise 0,9 mm.

Nr. 3) ist vorzugsweise ein Sieb mit einer glatten Oberfläche und Sieböffnungen mit einem Durchmesser von 0,18 bis 0,6 mm und vorzugsweise 0,4 mm.

Nr. 4) ist ein von einer Sieblochscheibe mit vorzugsweise Sieböffnungen vom Durchmesser 0,3 bis 1,0 mm getragenes, vorzugsweise aus Polycarbonat bestehendes transparentes Membransieb, dessen Sieböffnungen einen Durchmesser von 8 bis 30 $\mu$m und vorzugsweise 12 $\mu$m aufweisen.

Die Abtrennung der Trichinen lässt sich statt über Vakuum oder zusätzlich zum Vakuum auch unter Zuhilfenahme von Druckluft vornehmen, die bei geschlossenen Einlassventilen 15, 16 und 17 und bei geschlossener Deckelöffnung 12 durch das Ventil 11 eingeleitet wird.

(f) Nach vollständiger Passage des flüssigen Teils des Ansatzes durch die Filter werden die Ventile 11, 2 und 14 der Reihe nach geschlossen und der Reaktor mit 150 ml mit HCl auf pH 1,1 gebrachtem Leitungswasser durch die Sprühdüse 17 nachgespült. Anschliessend wird das Spülwasser durch Öffnen der genannten Ventile 11, 2 und 14 über Vakuum durch die Separiervorrichtung abgeführt und so auch ein Nachspülen der Separiervorrichtung erreicht.

(g) Zum Nachweis der Trichinen wird die Polycarbonat-Membran nach Abkoppeln der Separiervorrichtdung dieser entnommen, mit der Sedimentseite nach unten möglichst luftblasenfrei auf eine mit 2 bis 3 Tropfen Leitungswasser beträufelte Glasplatte aufgelegt und nach dem Beträufeln mit 2 bis 3 Tropfen Wasser möglichst luftblasenfrei mit einer zweiten Glasplatte abgedeckt und mikroskopisch auf das Vorhandensein von Trichinen untersucht.

Die Separiervorrichtung wird anschliessend mit den Sieben der genannten Beschaffenheit und Reihenfolge beschickt, die nach ihrer Benutzung in einem früheren Verfahrensgang 2 bis 24 h oder auch länger in einer 5%igen Pepsinlösung vom pH 1,2 gereinigt, danach mit scharfem Wasserleitungsstrahl abgespült und gegebenenfalls zur Bevorratung getrocknet worden sind.

Eine von dieser Verfahrensweise zum Teil abweichende Verfahrensweise besteht darin, dass die mechanische und chemische Zerkleinerung gemäss (a) bis (d) nach Anlegen eines nicht zu hohen Vakuuums im Reaktor und dessen hermetischem Abschluss gegenüber der Atmosphäre erfolgt. Dieses Vorgehen hat den Vorteil, dass kein oder nur sehr wenig Schaum entsteht und somit auf die Zugabe von Octanol verzichtet werden kann. Für das Brechen von Schaum ausreichend und aus technischen Gründen von Vorteil ist auch eine Verfahrensweise, bei der unter atmosphärischem Druck zerkleinert, jedoch zum Brechen des Schaums unmittelbar vor dem Abtrennen im Reaktor ein momentanes Vakuum angelegt und nach seiner unmittelbaren Beseitigung sofort abgetrennt wird.

## Zweites bevorzugtes Verfahren

Ein sehr stark vereinfachtes Verfahren zum Nachweis von Trichinen mit der erfindungsgemässen Apparatur besteht in folgendem:

Die auf 46°C vorerwärmte Apparatur wird durch die Deckelöffnung 12 mit 30 g Probenmaterial und 100 ml einer 5%igen Pepsinlösung beschickt und anschliessend 3 Minuten bei einer Messerdrehzahl von 12.000 UpM zerkleinert. Nach Abschalten des Motors werden durch die Deckelöffnung 12 mit einem Messbecher 400 ml einer 0,75%igen Salzsäurelösung zugegeben und die Deckelöffnung verschlossen. Danach wird der Ansatz erneut 1 Minute bei einer Messerdrehzahl von 12.000 UpM und im folgenden 7 Minuten bei einer Messerdrehzahl von 1.000 bis 2.000 UpM gerührt. Anschliessend wird die Deckelöffnung 12 geöffnet, 0,5 ml Octanol zugegeben, der Motor sofort abgeschaltet und nach Stillstand des Drehmessers die Ablassventile 2 und 14 geöffnet und der flüssige Anteil des Ansatzes über Vakuum abgetrennt.

Nach erfolgter Abtrennung werden die Ventile 14, 2, nach Zugabe von 150 ml HCl-Lösung von pH 1,1 auch die Deckelöffnung 12 geschlossen, das Drehmesser etwa 1 Sekunde auf 12.000 UpM gebracht, dann sofort abgeschaltet und das Spülwasser über Vakuum durch die Siebe abgeführt.

Anstatt über Vakuum oder zusätzlich zum Vakuum kann auch bei dieser vereinfachten Verfahrensweise unter Zuhilfenahme von Druckluft aufgetrennt werden.

## Drittes bevorzugtes Verfahren mit einer verkleinerten und vereinfachten Apparatur für die Anwendung bei Einzelschlachtungen oder kleiner Schlachtzahl und ausserhalb von Laboratorien

Die Verkleinerung der Apparatur ist dadurch gekennzeichnet, dass der Reaktorbecher ein Inhaltsvolumen von nur ca. 250 ml aufweist und die Vereinfachung erstens dadurch, dass eine der Thermostatisierung dienende Ummantelung des Reaktors nicht vorhanden ist; zum zweiten dadurch, dass der über einen Bajonettverschluss mit dem Reaktorbecher luftdicht verschliessbare Reaktordeckel nur mit einem Ventil 11 zum Einpumpen von Luft mittels Gummiball zwecks Erzeugung eines Überdruckes von wenigstens 1 bar ausgestattet ist und das Ventil 11 ein Rückschlagventil ist; zum dritten vorzugsweise dadurch, dass der Motor das abgestumpfte Drehmesser mit einer nicht regulierbaren, im Leerlauf konstanten Drehgeschwindigkeit von vorzugsweise 6.000 bis 12.000 UpM dreht und mit einer variabel einstellbaren, automatischen Intervallschaltung versehen ist. Diese Intervallschaltung ermöglicht einen Wechsel von einer halben Minute bis 5 Minuten Betrieb und einer halben Minute bis 5 Minuten Stillstand.

Diese Apparatur wird folgenderweise benutzt: Der Reaktor wird mit einer Probe von 5 bis 10 g, vorzugsweise 7 g Skelettmuskulatur und mittels eines Messlöffels mit 1,5 g Pepsin sowie mittels eines Messbechers mit 130 ml einer 0,5%igen HCl-Lösung von Zimmertemperatur bis 48°C und vorzugsweise 48°C beschickt.

Sofern es sich um eine Einzelschlachtung handelt, besteht die Probe aus einem einzigen etwa wallnussgrossen Stück Zwerchfellpfeiler. Bei zwei bzw. drei Schweinen handelt es sich um etwa halbwallnussgrosse bzw. kirschgrosse Skelettmuskelstücke. Bei 4 bis 10 zu untersuchenden Schweinen besteht die Sammelprobe aus einer der Zahl der Schlachtschweine entsprechenden Zahl gut haselnussgrosser Einzelproben.

Nachdem das Pepsin mit einer Spatel in der 0,5%igen HCl verrührt wurde, wird der Reaktordeckel luftdicht geschlossen und der Ansatz 2 Minuten zerkleinert. Anschliessend wird der Motor auf Intervallschaltung geschaltet und der Ansatz im Wechsel von jeweils 1 Minute Betrieb und jeweils 2 Minuten Stillstand insgesamt weitere 5 Minuten behandelt. Danach wird abgeschaltet, im Reaktor mit dem Gummiball ein Überdruck von wenigstens 1 bar erzeugt und danach das Auslassventil 2 geöffnet. Nach Passage des flüssigen Teils des Ansatzes durch die Separiervorrichtung wird der Reaktordeckel abgenommen, mit 0,5%iger HCl aus der Spritzflasche in den Reaktorbecher abgespült und nach Abspülen auch der Reaktorinnenfläche mit dem Reaktorbecher luftdicht verschlossen. Anschliessend wird mit dem Gummiball erneut ein Überdruck von wenigstesn 1 bar erzeugt und danach erneut abgetrennt. Der Nachweis der Trichinen erfolgt wie oben beschrieben.

Anstelle der 130 ml 0,5%ige HCl-Lösung kann ebenso eine Pufferlösung vom pH 1,1 verwendet werden.

**Patentansprüche**
**Patentansprüche für folgende Vertragsstaaten:**
**BE, CH, DE, FR, GB, IT, LI, NL, SE**

1.  Vorrichtung zur Erzeugung bzw. Freisetzung und Abtrennung von Substanzen oder partikulären Gebilden aus flüssiger, plastischer oder fester Materie, **gekennzeichnet** durch einen sich nach unten verjüngenden Reaktor (1), welcher in seinem unteren Teil ein Ablassventil (2) aufweist und über ein sich konisch erweiterndes Teil (3) mit einer nachgeschalteten Separiervorrichtung (4) verbunden ist, wobei die Separiervorrichtung (4) einen zylindrischen Anschlussstutzen (5) und ein weiteres Teil (21) umfasst, und der Anschlussstutzen (5) Halteringe (6) zur Halterung von einem oder mehreren Filtern bzw. Sieben (7) aufnimmt, und sich in einem lösbaren Kontakt mit dem sich konisch erweiternden Teil und/oder dem weiteren Teil (21) befindet."

2.  Vorrichtung gemäss Anspruch 1, dadurch **gekennzeichnet**, dass der Reaktor (1) in seinem mittleren Teil einen flüssigkeits- bzw. gasdichten Mantel (10) aufweist oder mit einem elektrischen Heizband, sowie mit einer oder mehreren Schrauböffnungen (19,20) und/oder einem Temperaturfühler, und/oder einer ionensensitiven Elektrode und/oder einem Leitfähigkeitsfühler ausgestattet ist.

3.  Vorrichtung gemäss Anspruch 1, dadurch **gekennzeichnet**, dass der Reaktor (1) mit einem Deckelteil (8) ausgestattet ist, und in demselben und/oder in der Wandung des Reaktors (1) ein oder mehrere Ventile zur Druckveränderung im Reaktor aufweist.

4.  Vorrichtung gemäss Anspruch 1, dadurch **gekennzeichnet**, dass der Reaktor (1) Öffnungen (12) oder Leitungen zum Beschicken desselben mit Probengut bzw. mit Flüssigkeit bzw. zur Be- oder Entgasung aufweist.

5.  Vorrichtung gemäss Anspruch 1, dadurch **gekennzeichnet**, dass durch den Deckelteil (8) oder die Wandung des Reaktors (1) der Schaft einer Zerkleinerungsvorrichtung (9) ragt.

6.  Vorrichtung gemäss Anspruch 1, dadurch **gekennzeichnet**, dass die Separiervorrichtung (4) über den Anschlussstutzen (5) durch einen Spann- oder Bajonett- oder Gewindeverschluss mit dem Teil (3) verbunden ist.

7.  Vorrichtung gemäss Anspruch 1, dadurch **gekennzeichnet**, dass ein oder mehrere Siebe (7) der Separiervorrichtung (4) Membransiebe oder Filter sind, die jeweils von einer Sieblochscheibe getragen werden.

8.  Vorrichtung gemäss Anspruch 1, dadurch **gekennzeichnet**, dass das dem Ablassventil nächstgelegene Filter aus einem zu einer glatten Oberfläche verschweissten Kunststoff- oder Metallgeflecht mit rautenförmigen Sieböffnungen mit einem Längsdurchmesser von 1,0 bis 2,0 mm und einem Querdurchmesser von 0,7 bis 1,4 mm besteht, das in Durchflussrichtung an zweiter Stelle liegende Filter aus einem zu einer glatten Oberfläche verschweissten Kunststoff- oder Metallgeflecht mit rautenförmigen Sieböffnungen mit einem Durchmesser von 0,3 bis 1,2 mm, das in Durchflussrichtung an dritter Stelle liegende Filter aus einem Sieb mit Sieböffnungen von einem Durchmesser von 0,18 bis 0,6 mm besteht, und das in Durchflussrichtung an vierter Stelle liegende Sieb ein von einer Sieblochscheibe mit Sieböffnungen vom Durchmesser 0,3 bis 1,0 mm getragenes, vorzugsweise aus Polycarbonat bestehendes transparentes Membransieb ist, dessen Sieböffnungen einen Durchmesser von weniger als 0,18 mm aufweisen.

9.  Verfahren zur Erzeugung bzw. Freisetzung und Abtrennung von Substanzen oder partikulären Gebilden aus plastischer oder fester Materie unter Verwendung der Vorrichtung gemäss Anspruch 1, dadurch **gekennzeichnet**, dass man das plastische oder feste Material zusammen mit einer Flüssigkeit in einem Reaktor einer chemischen und/oder physikalischen Behandlung unterwirft, das erhaltene Gemisch über das sich im unteren Teil des Reaktors befindliche Ablassventil einer Separiervorrichtung zuführt, in welcher mit Hilfe eines oder mehrerer Siebe und/oder Membransiebe und/oder Filter eine Auftrennung desselben in eine flüssige und eine oder mehrere feste Phasen vorgenommen wird.

10. Verfahren gemäss Anspruch 9, dadurch **gekennzeichnet**, dass die Auftrennung in der Separiervorrichtung mit Hilfe von ein bis vier Sieben und/oder Membransieben und/oder Filtern erfolgt, auf denen Partikel unterschiedlicher Grösse zurückgehalten werden, und die Porosität der Siebe und/oder Membransiebe und/oder Filter in Durchflussrichtung abnimmt.

11. Verfahren gemäss Anspruch 9, dadurch **gekennzeichnet**, dass zur Auftrennung in der Separiervorrichtung ein Satz aus mehreren transparenten Membransieben mit Sieböffnungen vom Durchmesser bis zu 180 $\mu$m, deren Porengrösse in Durchflussrichtung abnimmt, verwendet wird.

12. Verfahren gemäss Anspruch 9, dadurch **gekennzeichnet**, dass die physikalische Behandlung der genannten Materie im Reaktor mit Hilfe eines an seiner Oberfläche profilierten Rotors, der innerhalb eines entsprechend profilierten Stators von einem sich ausserhalb des Reaktors befindlichen Motor angetrieben wird, durch Ultrabeschallung oder/und durch Einwirkung von Enzymen erfolgt.

13. Verfahren zur Freisetzung und Abtrennung von Trichinen aus Schweinefleisch unter Verwendung der Vorrichtung gemäss Anspruch 1, dadurch **gekennzeichnet**, dass ein ca. walnussgrosses Stück aus dem Zwerchfellpfeiler entnommenes Schweinefleisch im Reaktor unter Zugabe von 130 ml 0,5%iger HCl oder einer Pufferlösung von pH 1,1 mit Hilfe eines abgestumpften Drehmessers bei einer Umdrehungsgeschwindigkeit von vorzugsweise 12 x $10^3$ UpM zerkleinert wird, das erhaltene Gemisch durch Zugabe von 30 ml 5%iger Pepsinlösung (pH 5,5) unter Freisetzung der Trichinen enzymatisch behandelt wird, das enzymatisch behandelte Gemisch über das Ablassventil auf die Separiervorrichtung gegeben wird, eine Auftrennung in eine flüssige und feste Phase über einem Sieb mit Öffnungen vom Durchmesser 1,0 bis 1,5 mm, einem in Durchflussrichtung nachfolgenden Sieb mit Öffnungen von 0,6 mm und einem nachfolgenden transparenten Membransieb mit Poren vom Durchmesser 12 bis 30 $\mu$m erfolgt, und die auf dem 12 bis 30 $\mu$m-Filter abgelagerten Trichinen unmittelbar unter dem Lichtmikroskop untersucht werden .

**Patentansprüche für folgenden Vertragsstaat: AT**

1. Verfahren zur Erzeugung bzw. Freisetzung und Abtrennung von Substanzen oder partikulären Gebilden aus flüssiger, plastischer oder fester Materie, dadurch **gekennzeichnet**, dass man das plastische oder feste Material zusammen mit einer Flüssigkeit in einem Reaktor einer chemischen und/oder physikalischen Behandlung unterwirft, das erhaltene Gemisch über das sich im unteren Teil des Recktors befindliche Ablassventil einer Separiervorrichtung zuführt, in welcher mit Hilfe eines oder mehrerer Siebe und/oder Membransiebe und/oder Filter eine Auftrennung desselben in eine flüssige und eine oder mehrere feste Phasen vorgenommen wird.

2. Verfahren gemäss Anspruch 1, dadurch **gekennzeichnet**, dass die Auftrennung in der Separiervorrichtung mit Hilfe von ein bis vier Sieben und/oder Membransieben und/oder Filtern erfolgt, auf denen Partikel unterschiedlicher Grösse zurückgehalten werden, und die Porosität der Siebe und/oder Membransiebe und/oder Filter in Durchflussrichtung abnimmt.

3. Verfahren gemäss Anspruch 1 oder 2, dadurch **gekennzeichnet**, dass zur Auftrennung in der Separiervorrichtung ein Satz aus mehreren transparenten Membransieben mit Sieböffnungen vom Durchmesser bis zu 180 $\mu$m, deren Porengrösse in Durchflussrichtung abnimmt, verwendet wird.

4. Verfahren gemäss Anspruch 1 bis 3, dadurch **gekennzeichnet**, dass die physikalische Behandlung der genannten Materie im Reaktor mit Hilfe eines an seiner Oberfläche profilierten Rotors, der innerhalb eines entsprechend profilierten Stators von einem sich ausserhalb des Recktors befindlichen Motor angetrieben wird, durch Ultrabeschallung oder/und durch Einwirkung von Enzymen erfolgt.

5. Verfahren zur Freisetzung und Abrennung von Trichinen aus Schweinefleisch, dadurch **gekennzeichnet**, dass ein ca. walnussgrosses Stück aus dem Zwerchfellpfeiler entnommenes Schweinefleisch im Reaktor unter Zugabe von 130 ml 0,5%iger HCl oder einer Pufferlösung von pH 1,1 mit Hilfe eines abgestumpften Drehmessers bei einer Umdrehungsgeschwindigkeit von vorzugsweise 12 x $10^3$ UpM zerkleinert wird, das erhaltene Gemisch durch Zugabe von 30 ml 5%iger Pepsinlösung (pH 5,5) unter Freisetzung der Trichinen enzymatisch behandelt wird, das enzymatisch behandelte Gemisch über das Ablassventil auf die Separiervorrichtung gegeben wird, eine Auftrennung in eine flüssige und feste Phase über einem Sieb mit Öffnungen vom Durchmesser 1,0 bis 1,5 mm, einem in Durchflussrichtung nachfolgenden Sieb mit Öffnungen von 0,6 mm und einem nachfolgenden transparenten Membransieb mit Poren vom Durchmesser 12 bis 30 $\mu$m erfolgt, und die auf dem 12 bis 30 $\mu$m-Filter abgelagerten Trichinen unmittelbar unter dem Lichtmikroskop untersucht werden .

6. Verfahren nach einem oder mehreren der vorstehenden Ansprüche 1 bis 5, dadurch **gekennzeichnet**, dass die Siebe bzw. Filter in der Separiervorrichtung wie folgt gewählt werden:
   - das dem Ablassventil nächstgelegene Filter besteht aus einem zu einer glatten Oberfläche veschweissten Kunststoff- oder Metallgeflecht mit rautenförmigen Sieböffnungen mit einem Längsdurchmesser von 1,0 bis 2,0 mm und einem Querdurchmesser von 0,7 bis 1,4 mm;
   - das in Durchflussrichtung an zweiter Stelle liegende Filter besteht aus einem zu einer glatten Oberfläche verschweissten Kunststoff- oder Metallgeflecht mit rautenförmigen Sieböffnungen mit einem Durchmesser von 0,3 bis 1,2 mm;
   - das in Durchflussrichtung an dritter Stelle liegende Filter besteht aus einem Sieb mit Sieböffnungen von einem Durchmesser von 0,18 bis 0,6 mm; und
   - das in Durchflussrichtung an vierter Stelle liegende Sieb besteht aus einem von einer Sieblochscheibe mit Sieböffnungen vom Durchmesser 0,3 bis 1,0 mm getragenen, vorzugsweise aus Polycarbonat bestehendem transparentem Membransieb, dessen Sieböffnungen einen Durchmesser von weniger als 0,18 mm aufweisen.

## Claims

**Claims for the following Contracting States: BE, CH, DE, FR, GB, IT, LI, NL, SE**

1. A device for producing and for releasing and separating substances or particulate structures of liquid, plastic or solid materials, characterised by a downwardly tapering reactor (1) which in its lower part comprises an outlet valve (2) and is connected via a conically widening component (3) to a following separating device (4), wherein the separating device (4) comprises a cylindrical connection piece (5) and a further component (21), and the connection piece (5) accommodates retaining rings (6) which support one or more filters or sieves (7), and forms a detachable contact with the conically widening component and/or the further component (21).

2. A device as claimed in Claim 1, characterised in that in its central part the reactor (1) is provided with a liquid- and gas-tight casing (10) or is equipped with an electric heating strip, and with one or more screw openings (19, 20) and/or a temperature sensor, and/or an ion-sensitive electrode, and/or a conductivity sensor.

3. A device as claimed in Claim 1, characterised in that the reactor (1) is equipped with a cover component (8) and in the cover component and/or in the walls of the reactor (1) comprises one or more valves for changing the pressure in the reactor.

4. A device as claimed in Claim 1, characterised in that the reactor (1) comprises openings (12) or lines by which it is supplied with sample material and with liquid and via which gas is supplied or eliminated.

5. A device as claimed in Claim 1, characterised in that the shaft of a pulverising device (9) projects through the cover component (8) or the walls of the reactor (1).

6. A device as claimed in Claim 1, characterised in that the separating device (4) is connected via the connection piece (5) to the component (3) by a clamp- or bayonet- or screw joint.

7. A device as claimed in Claim 1, characterised in that one or more sieves (7) of the separating device (4) are membrane sieves or filters which are in each case supported by a perforated plate.

8. A device as claimed in Claim 1, characterised in that the filter arranged closest to the outlet valve is composed of a synthetic resin or metal mesh which is welded so as to form a smooth surface and which contains rhomboid filter openings with a longitudinal diameter of 1.0 to 2.0 mm and a transverse diameter of 0.7 to 1.4 mm, the filter arranged in the second position, considered in the direction of flow, is composed of a synthetic resin or metal mesh which is welded so as to form a smooth surface and which contains rhomboid filter openings with a diameter of 0.3 to 1.2 mm, the filter arranged in the third position, considered in the direction of flow, comprises a sieve containing openings with a diameter of 0.18 to 0.6 mm, and the filter arranged in the fourth position, considered in the direction of flow, comprises a transparent membrane sieve which is preferably made of polycarbonate, the openings of which have a diameter of less than 0.18 mm, and which is supported by a perforated plate itself containing openings with a diameter of 0.3 to 1.0 mm.

9. A process for the production and for the release and separation of substances or particulate structures of plastic or solid materials using the device claimed in Claim 1, characterised in that the plastic or solid material, together with a liquid, is subjected to a chemical and/or physical treatment in a reactor, the obtained mixture is fed, via the outlet valve arranged in the lower part of the reactor, to a separating device in which, with the aid of one or more sieves and/or membrane sieves and/or filters, the mixture is separated into a liquid phase and one or more solid phases.

10. A process as claimed in Claim 9, characterised in that the separation in the separating device is carried out using one to four sieves and/or membrane sieves and/or filters on which particles of different sizes are retained, and the porosity of the sieves and/or membrane sieves and/or filters reduces in the direction of flow.

11. A process as claimed in Claim 9, characterised in that a set comprising a plurality of transparent membrane sieves containing openings with a diameter of up to 180 $\mu$m, where the pore size decreases in the direction of flow, is used for the separation in the separating device.

12. A process as claimed in Claim 9, characterised in that the physical processing of the aforementioned materials in the reactor is carried out using a rotor, which is profiled at its surface and which is driven within a correspondingly profiled stator by a motor arranged outside the reactor, by means of ultrasonic and/or enzymatic treatment.

13. A process for releasing and separating trichinae from pork using the device claimed in Claim 1, characterised in that an approximately walnut sized piece of pork taken from the diaphragm muscle is pulverised in the reactor, with the addition of 130 ml 0.5% HCl or a buffer solution of pH 1.1, using a blunted rotating blade at a rotation speed of preferably 12 x $10^3$ UpM, the obtained mixture is treated enzymatically by the addition of 30 ml 5% pepsin solution (pH 5.5) whereby the trichinea are released, the enzymatically treated mixture is fed via the outlet valve to the separating device, separation into a liquid phase and a solid phase is carried out using a sieve containing openings with a diameter of 1.0 to 1.5 mm, a sieve arranged downstream in the direction of flow containing openings with a diameter of 0.6 mm, and a transparent membrane sieve arranged downstream in the direction of flow and containing pores with a diameter of 12 to 30 $\mu$m, and the trichinae deposited on the 12 to 30 $\mu$m filter are immediately investigated under an optical microscope.

**Claims for the following Contracting State: AT**

1. A process for producing and for releasing and separating substances or particulate structures of liquid, plastic or solid materials, characterised in that the plastic or solid material, together with a liquid, is subjected to a chemical and/or physical treatment in a reactor, the obtained mixture is fed, via the outlet valve arranged in the lower part of the reactor, to a separating device in which, with the aid of one or more sieves and/or membrane sieves and/or filters, the mixture is separated into a liquid phase and one or more solid phases.

2. A process as claimed in Claim 1, characterised in that the separation in the separating device is carried out by means of one to four sieves and/or membrane sieves and/or filters, on which particles of different sizes are retained, and the porosity of the sieves and/or membrane sieves and/or filters reduces in the direction of flow.

3. A process as claimed in Claim 1 or 2, characterised in that a set comprising a plurality of transparent membrane sieves containing openings with a diameter of up to 180 $\mu$m, where the pore size reduces in the direction of flow, is used for the separation in the separating device.

4. A process as claimed in Claim 1 to 3, characterised in that the physical processing of the aforementioned materials in the reactor is carried out using a rotor, which is profiled at its surface and which is driven within a correspondingly profiled stator by a motor arranged outside the reactor, by means of ultrasonic and/or enzymatic treatment.

5. A process for releasing and separating trichinae from pork, characterised in that an approximately walnut sized piece of pork taken from the diaphragm muscle is pulverised in the reactor, with the addition of 1.30 ml 0.5% HCl or a buffer solution of pH 1.1, using a blunted rotary blade at a rotation speed of preferably $12 \times 10^3$ UpM, the obtained mixture is enzymatically treated by the addition of 30 ml 5% pepsin solution (pH 5.5) whereby the trichinae are released, the enzymatically treated mixture is fed via the outlet valve to the separating device, separation into a liquid phase and a solid phase is carried out using a sieve containing openings with a diameter of 1.0 to 1.5 mm, a sieve arranged downstream in the direction of flow and containing openings with a diameter of 0.6 mm, and a transparent membrane sieve arranged downstream in the direction of flow and containing pores with a diameter of 12 to 30 $\mu$m, and the trichinae deposited on the 12 to 30 $\mu$m filter are immediately investigated under an optical microscope.

6. A process as claimed in one or more of the preceding Claims 1 to 5, characterised in that the sieves or filters in the separating device are selected as follows:
- the filter arranged closest to the outlet valve is composed of a synthetic resin or metal mesh which is welded so as to form a smooth surface and which contains rhomboid filter openings with a longitudinal diameter of 1.0 to 2.0 mm and a transverse diameter of 0.7 to 1.4 mm;
- the filter arranged in the second position considered in the direction of flow is composed of a synthetic resin or metal mesh which is welded so as to form a smooth surface and which contains rhomboid filter openings with a diameter of 0.3 to 1.2 mm;
- the filter arranged in the third position considered in the direction of flow comprises a sieve containing openings with a diameter of 0.1 to 0.6 mm and
- the filter arranged in the fourth position in the direction of flow comprises a transparent membrane sieve which is preferably made of polycarbonate, the openings of which have a diameter of less than 0.18 mm, and which is supported by a perforated plate itself containing openings with a diameter of 0.3 to 1.0 mm.

**Revendications**
**Revendications pour les Etats contractants suivants: BE, CH, DE, FR, GB, IT, LI, NL, SE**

1. Dispositif pour former ou libérer et séparer des substances ou des produits particulaires à partir d'une matière liquide, plastique ou solide, caractérisé par un réacteur (1) qui va en se rétrécissant vers le bas, qui présente dans sa partie inférieur une soupape de décharge (2) et qui est relié à un dispositif séparateur (4) branché en aval par l'intermédiaire d'une partie (3) qui s'évase de manière conique, le dispositif séparateur (4) comportant une tubulure de raccordement (5) cylindrique et une autre partie (21), et la tubulure de raccordement (5) contenant des bagues de soutien (6) pour soutenir un ou plusieurs filtres ou tamis (7), et se trouvant en contact amovible avec la partie qui s'évase de manière conique et/ou l'autre partie (21).

2. Dispositif selon la revendication 1, caractérisé en ce que le réacteur (1) présente dans sa partie moyenne une enveloppe (10) étanche aux liquides ou aux gaz ou est muni d'une bande chauffante électrique, ainsi que d'une ou plusieurs ouvertures à vis (19, 20) et/ou d'un capteur de températures, et/ou d'une électrode sensible aux ions et/ou d'un détecteur de conductivité.

3. Dispositif selon la revendication 1, caractérisé en ce que le réacteur (1) est muni d'une partie formant couvercle (8) et comporte dans celle-ci et/ou dans la paroi du réacteur (1) une ou plusieurs vannes pour modifier la pression dans le réacteur.

4. Dispositif selon la revendication 1, caractérisé en ce que le réacteur (1) présente des ouvertures (12) ou des conduites pour introduire dans celui-ci les échantillons ou un liquide ou pour introduire ou évacuer un gaz.

5. Dispositif selon la revendication 1, caractérisé en ce que l'arbre d'un dispositif de broyage (9) traverse la partie formant couvercle (8) ou la paroi du réacteur (1).

6. Dispositif selon la revendication 1, caractérisé en ce que le dispositif séparateur (4) est relié à la partie (3) par un joint à tension, à baïonnette ou à vis par l'intermédiaire de la tubulure de raccordement (5).

7. Dispositif selon la revendication 1, caractérisé en ce qu'un ou plusieurs tamis (7) du dispositif séparateur (4) sont des tamis ou filtres à membrane portés chacun par un disque perforé de tamis.

8. Dispositif selon la revendication 1, caractérisé en ce que le filtre le plus proche de la soupape de décharge consiste en un treillis en matière plastique ou en métal, soudé à une surface lisse, qui présente des ouvertures de tamis en forme de losange ayant un diamètre longitudinal de 1,0 à 2,0 mm et un diamètre transversal de 0,7 à 1,4 mm, le filtre situé en seconde position dans la direction de l'écoulement consiste en un treillis en matière plastique ou en métal, soudé à une surface lisse, comportant des ouvertures de tamis en forme de losange ayant un diamètre de 0,3 à 1,2 mm, le filtre situé en troisième position dans la direction de l'écoulement consiste en un tamis comportant des ouvertures ayant un diamètre de 0,18 à 0,6 mm, et le tamis situé en quatrième position dans la direction de l'écoulement est un tamis à membrane transparent consistant de préférence en polycarbonate qui est porté par un disque perforé de tamis présentant des ouvertures d'un diamètre de 0,3 à 1,0 mm, tamis à membrane dont les ouvertures de tamis présentent un diamètre inférieur à 0,18 mm.

9. Procédé de formation ou de libération et séparation de substances ou de produits particulaires à partir d'une matière plastique ou solide à l'aide du dispositif selon la revendication 1, caractérisé en ce que l'on soumet la matière plastique ou solide et un liquide à un traitement chimique et/ou physique dans un réacteur, on amène le mélange obtenu par l'intermédiaire d'une soupape de décharge située dans la partie inférieure du réacteur à un dispositif séparateur dans lequel s'effectue une séparation de celui-ci à l'aide d'un ou plusieurs tamis et/ou tamis à membrane et/ou filtres en une phase liquide et en une ou plusieurs phases solides.

10. Procédé selon la revendication 9, caractérisé en ce que la séparation dans le dispositif séparateur a lieu à l'aide d'un à quatre tamis et/ou tamis à membrane et/ou filtres sur lesquels sont retenues des particules de tailles différentes, et la porosité des tamis et/ou tamis à membrane et/ou filtres décroît dans la direction de l'écoulement.

11. Procédé selon la revendication 9, caractérisé en ce que, pour la séparation dans le dispositif séparateur, on utilise un jeu de plusieurs tamis à membrane transparents présentant des ouvertures de tamis d'un diamètre atteignant 180 μm dont la taille des pores décroît dans la direction d'écoulement.

12. Procédé selon la revendication 9, caractérisé en ce que le traitement physique de la matière citée dans le réacteur a lieu à l'aide d'un rotor profilé à sa surface qui est entraîné à l'intérieur d'un stator profilé de manière correspondante par un moteur situé à l'extérieur du réacteur, par ultrasons et/ou par action d'enzymes.

13. Procédé de libération et de séparation de trichines de la viande de porc au moyen du dispositif selon la revendication 1, caractérisé en ce qu'un fragment sensiblement de la taille d'une noix de viande de porc prélevée sur les piliers du diaphragme est broyé dans le réacteur avec addition de 130 ml de HCl à 0,5 % ou d'une solution tampon d'un pH de 1,1 à l'aide d'un couteau rotatif émoussé à une vitesse de rotation de préférence de $12 \times 10^3$ tr/min, le mélange obtenu est traité par voie enzymatique par addition de 30 ml d'une solution de pepsine à 5 % (pH 5,5) avec libération des trichines, le mélange traité par voie enzymatique est appliqué par l'intermédiaire de la soupape de décharge sur le dispositif séparateur, il se produit une séparation en une phase liquide et une phase solide sur un tamis présentant des ouvertures d'un diamètre de 1,0 à 1,5 mm, un tamis suivant dans la direction de l'écoulement présentant des ouvertures de 0,6 mm et un tamis a membrane transparent suivant comportant des pores d'un diamètre de 12 à 30 μm, et les trichines déposées sur le filtre de 12 à 30 μm sont examinées immédiatement au microscope optique.

**Revendications pour l'Etat contractant suivant: AT**

1. Procédé de formation ou de libération et séparation de substances ou de produits particulaires à partir d'une matière liquide, plastique ou solide, caractérisé en ce que l'on soumet la matière plastique ou solide et un liquide à un traitement chimique et/ou physique dans un réacteur, on amène le mélange obtenu par l'intermédiaire d'une soupape de décharge située dans la partie inférieure du réacteur à un dispositif séparateur dans lequel s'effectue une séparation de celui-ci à l'aide d'un ou plusieurs tamis et/ou tamis à membrane et/ou filtres en une phase liquide et en une ou plusieurs phases solides.

2. Procédé selon la revendication 1, caractérisé en ce que la séparation dans le dispositif séparateur a lieu à l'aide d'un à quatre tamis et/ou tamis à membrane et/ou filtres sur lesquels sont retenues des particules de tailles différentes, et la porosité des tamis et/ou tamis à membrane et/ou filtres décroît dans la direction de l'écoulement.

3. Procédé selon la revendication 1 ou 2, caractérisé en ce que, pour la séparation dans le dispositif séparateur, on utilise un jeu de plusieurs tamis à membrane transparents présentant des ouvertures de tamis d'un diamètre atteignant 180 $\mu$m dont la taille des pores décroît dans la direction d'écoulement.

4. Procédé selon les revendications 1 à 3, caractérisé en ce que le traitement physique de la matière citée dans le réacteur a lieu à l'aide d'un rotor profilé à sa surface qui est entraîné à l'intérieur d'un stator profilé de manière correspondante par un moteur situé à l'extérieur du réacteur, par ultrasons et/ou par action d'enzymes.

5. Procédé de libération et de séparation de trichines de la viande de porc, caractérisé en ce qu'un fragment sensiblement de la taille d'une noix de viande de porc prélevée sur les piliers du diaphragme est broyé dans le réacteur avec addition de 130 ml de HCl à 0,5 % ou d'une solution tampon d'un pH de 1,1 à l'aide d'un couteau rotatif émoussé à une vitesse de rotation de préférence de 12 x 10³ tr/min, le mélange obtenu est traité par voie enzymatique par addition de 30 ml d'une solution de pepsine à 5 % (pH 5,5) avec libération des trichines, le mélange traité par voie enzymatique est appliqué par l'intermédiaire de la soupape de décharge sur le dispositif séparateur, il se produit une séparation en une phase liquide et une phase solide sur un tamis présentant des ouvertures d'un diamètre de 1,0 à 1,5 mm, un tamis suivant dans la direction de l'écoulement présentant des ouvertures de 0,6 mm et un tamis à membrane transparent suivant comportant des pores d'un diamètre de 12 à 30 $\mu$m, et les trichines déposées sur le filtre de 12 à 30 $\mu$m sont examinées immédiatement au microscope optique.

6. Procédé selon une ou plusieurs des revendications 1 à 5, caractérisé en ce que les tamis ou filtres du dispositif séparateur sont choisis de la manière suivante :

le filtre le plus proche de la soupape de décharge consiste en un treillis en matière plastique ou en métal, soudé à une surface lisse, qui présente des ouvertures de tamis en forme de losange ayant un diamètre longitudinal de 1,0 à 2,0 mm et un diamètre transversal de 0,7 à 1,4 mm, le filtre situé en seconde position dans la direction de l'écoulement consiste en un treillis en matière plastique ou en métal, soudé à une surface lisse, comportant des ouvertures de tamis en forme de losange ayant un diamètre de 0,3 à 1,2 mm, le filtre situé en troisième position dans la direction de l'écoulement consiste en un tamis comportant des ouvertures ayant un diamètre de 0,18 à 0,6 mm, et le tamis situé en quatrième position dans la direction de l'écoulement est un tamis à membrane transparent consistant de préférence en polycarbonate qui est porté par un disque perforé de tamis présentant des ouvertures d'un diamètre de 0,3 à 1,0 mm, tamis à membrane dont les ouvertures de tamis présentent un diamètre inférieur à 0,18 mm.

FIG. 1

FIG. 2

FIG. 3